(19) Europäisches Patentamt
European Patent Office
Office européen des brevets

(11) Veröffentlichungsnummer: **0 008 361**
**B1**

(12) # EUROPÄISCHE PATENTSCHRIFT

(45) Veröffentlichungstag der Patentschrift: **27.01.82**

(21) Anmeldenummer: **79102463.1**

(22) Anmeldetag: **16.07.79**

(51) Int. Cl.³: **C 07 D 333/28** // A01N57/16, A61K31/40, C07F9/165, C07D409/12

(54) **Halogensubstituierte Thiophen-Verbindungen und Verfahren zu ihrer Herstellung.**

(30) Priorität: **20.07.78 DE 2831871**

(43) Veröffentlichungstag der Anmeldung: **05.03.80 Patentblatt 80/5**

(45) Bekanntmachung des Hinweises auf die Patenterteilung: **27.01.82 Patentblatt 82/4**

(84) Benannte Vertragsstaaten: **BE CH DE FR GB**

(56) Entgegenhaltungen:
**DE-A-2 111 192**
**US-A-2 623 049**
**DE-A1-2 508 335**
**BEILSTEINS HANDBUCH DER ORGANISCHEN CHEMIE, 4. Auflage, 3. und 4. Ergänzungswerk, 7. Band, 1. Teil 1974, SPRINGER-VERLAG, Berlin, Heidelberg, New York, Seite 273, Mitte**

(73) Patentinhaber: **BASF Aktiengesellschaft, Carl-Bosch-Strasse 38, D-6700 Ludwigshafen (DE)**

(72) Erfinder: **Frickel, Fritz-Frieder, Dr. Chem., Prager Strasse 27, D-6700 Ludwigshafen (DE)**
Erfinder: **Wiersdorff, Walter-Wielant, Dr. Chem., An der Froschlache 23, D-6700 Ludwigshafen (DE)**

**EP 0 008 361 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Halogensubstituierte Thiophen-Verbindungen und Verfahren zu ihrer Herstellung

Gegenstand der vorliegenden Erfindung sind Thiophen-Verbindungen der Formel (I)

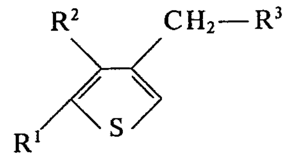

(I)

in der $R^1$ und $R^2$ jeweils gleich und Chlor- oder Bromatome und $R^3$ eine Hydroxygruppe oder ein Chlor- oder Bromatom bedeuten, und Verfahren zu ihrer Herstellung.

Als erfindungsgemäße Verbindungen können dementsprechend genannt werden:

2,3-Dichlor-4-hydroxymethyl-thiophen, 2,3-Dichlor-4-chlormethyl-thiophen, 2,3-Dichlor-4-brommethyl-thiophen, 2,3-Dibrom-4-hydroxymethylthiophen, 2,3-Dibrom-4-chlormethyl-thiophen, 2,3-Dibrom-4-brommethyl-thiophen.

Die erfindungsgemäßen Verbindungen werden aus bekannten Vorprodukten in an sich bekannter Weise durch Reduktions- und Substitutionsreaktionen hergestellt. Als Ausgangsverbindungen dienen halogenierte Thiophen-4-aldehyde der Formel II

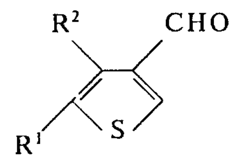

(II)

in der $R^1$ und $R^2$ die oben genannten Bedeutungen haben, wie sie beispielsweise von Gronowitz und Ander (Tetrahedron 32, 1403—1406 [1976]) beschrieben und durch Halogenieren von Thiophen-3-aldehyd hergestellt werden.

Insbesondere erfolgt die Herstellung der Verbindungen der Formel I, indem man einen Thiophenaldehyd der Formel II

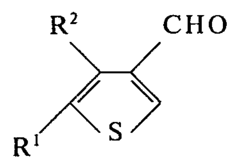

(II)

in der $R^1$ und $R^2$ die oben genannten Bedeutungen haben, mit einem komplexen Hydrid reduziert und gegebenenfalls die erhaltene Hydroxymethylverbindung mit einem Säurechlorid oder Säurebromid einer Schwefel- oder Phosphorsäure oder mit konzentrierter Chlorwasserstoff- oder Bromwasserstoffsäure umsetzt.

Geeignete komplexe Hydride sind Lithiumhydride oder Borhydride, beispielsweise $LiAlH_4$, $LiAl(OCH_3)_2H_2$, $NaBH_4$, $KBH_4$ oder andere Hydride vergleichbarer Reaktivität. Das bevorzugte Reduktionsmittel ist $NaBH_4$, da die Umsetzungen mit $NaBH_4$ auch in wäßrigen Lösungsmitteln durchgeführt werden können.

Die Reduktion wird zweckmäßigerweise in einem inerten Lösungsmittel durchgeführt. Im Fall von $NaBH_4$ werden niedere Alkohole, wie Methanol, Äthanol, Isopropanol oder Isobutanol, aber auch Dialkyläther oder gesättigte cyclische Äther, wie Diäthyläther, Methyl-tert.-butyläther, Dioxan, Tetrahydrofuran oder Kohlenwasserstoffe verwendet. Bevorzugt wird die Reaktion in Wasser oder in homogenen oder heterogenen Mischungen von Wasser mit einem der oben angegebenen Solventien oder beispielsweise mit Toluol, Cyclohexan, Pentan, Hexan durchgeführt, wenn $NaBH_4$ als Reduktionsmittel dient.

Das $NaBH_4$ wird gelöst oder in Suspension in Wasser oder in wäßrig-alkoholischer Lösung, wie Isopropanol-Wasser-Mischungen, verwendet.

Zweckmäßigerweise wird der Aldehyd der Formel II vorgelegt und das komplexe Hydrid zugegeben. Es kann aber auch in umgekehrter Reihenfolge verfahren werden.

Der zu reduzierende Aldehyd der Formel II kann an sich in beliebigen Konzentrationen gelöst, emulgiert oder suspendiert werden. Es hat sich jedoch als zweckmäßig erwiesen, den Ausgangsaldehyd in möglichst hohen Konzentrationen einzusetzen, ebenso wie das komplexe Hydrid. Wenn sich Probleme bezüglich der Viskosität oder der Phasentrennung ergeben, so kann zweckmäßigerweise während oder nach Ende der Reaktion verdünnt werden.

Die Menge des angewandten Hydrids entspricht in der Regel der äquimolaren Menge. Da die Umsetzung schnell und exotherm abläuft, läßt sich die genau benötigte Hydridmenge gegebenenfalls leicht durch einen Vorversuch ermitteln. Am einfachsten ist es jedoch, den zu reduzierenden halogenierten Thiophen-4-aldehyd der Formel II im Solvens vorzulegen und so lange $NaBH_4$ zuzugeben, wie die Reaktion exotherm verläuft. Ein rechnerischer Überschuß an $NaBH_4$ über 10% ist nur bei minderwertigem Hydrid oder stark verunreinigtem Aldehyd erforderlich.

Die Reduktion wird bei Temperaturen von −10°C bis 60°C durchgeführt, vorzugsweise zwischen 0 und 40°C.

Die Aufarbeitung des Reaktionsgutes kann in an sich bekannter Weise erfolgen, z. B. durch Zugabe von Essigester, Alkoholen, Wasser oder Mischungen von organischen Solventien mit Wasser. Im Falle des $NaBH_4$ als Reduktionsmittel wird nur so viel Wasser zugegeben, daß sich das entstandene 4-Hydroxymethyl-thiophen-derivat direkt abtrennen läßt. Die Phasentrennung kann durch organische Solventien unterstützt werden. Das entstandene 4-Hydroxymethylthiophen-derivat kann meist direkt weiterverwendet werden. Erscheint jedoch eine Reinigung nötig, so kann es vorzugsweise unter vermindertem Druck destillativ gereinigt werden.

Zur Herstellung der 4-Chlormethyl- oder 4-Brommethyl-thiophen-Verbindungen der Formel I versetzt man die entsprechenden 4-Hydroxymethyl-thiophene zweckmäßig in einem inerten Lösungsmittel in suspendierter, emulgierter oder gelöster Form mit einem Säurechlorid oder Säurebromid der Schwefel- oder Phosphorsäure, wie $SOCl_2$, $SOBr_2$, $PCl_3$, $PBr_3$ oder $POCl_3$, wie beispielsweise von Steinkopf und Eger in Ann. 533, 270, 273 (1938) beschrieben. Gegebenenfalls kann man einen Katalysator, beispielsweise ein tertiäres organisches Amin, wie Pyridin, Chinolin, Dimethylanilin, in Mengen von 0,01 bis 2 Mol pro Mol Ausgangsverbindung zusetzen.

Als inerte Lösungsmittel eignen sich vor allem aliphatische Halogenkohlenwasserstoffe, wie $CH_2Cl_2$, $C_2H_4Cl_2$ oder auch $CHCl_3$, gesättigte oder ungesättigte Kohlenwasserstoffe, wie n-Pentan, n-Hexan, Cyclohexan, aromatische Kohlenwasserstoffe, wie Toluol, Xylol oder Chlorbenzol. Gegebenenfalls kann ohne Solvens gearbeitet werden, wenn man beispielsweise einen Überschuß des Halogenierungsmittels als Lösungsmittel verwendet (vgl. auch Houben—Weyl 5/3, Seite 863, Georg Thieme Verlag, 1962).

Nach einer anderen Verfahrensweise werden konzentrierte, wäßrige oder nichtwäßrige Brom- oder Chlorwasserstoffsäuren verwendet. In diesem Fall genügt ein einfaches Ausführen des entsprechenden 4-Hydroxymethyl-thiophens mit konzentrierter Salzsäure oder Bromwasserstoffsäure bei Raumtemperatur, Verdünnen mit einem inerten Solvens, wie Methylenchlorid, und übliche Isolierung des erhaltenen 4-Halogenmethyl-thiophens.

Die erfindungsgemäßen Verbindungen sind wertvolle Zwischenprodukte für die Synthese pharmakologisch wirksamer Verbindungen, von Pflanzenschutzmitteln, sowie von Photoinitiatoren oder Aromastoffe. In diesem Zusammenhang wird beispielsweise auf die Veröffentlichtung von N. R. Clark in »Manufacturing Chemist and Aerosol News«, Juli 1972, S. 3—7, verwiesen.

Beispielsweise können durch weitere Umsetzung wiksame Diuretika hergestellt werden.

Insbesondere können durch Umsetzung mit Phosphorverbindungen Phosphorsäureester erhalten werden, die sich hervorragend zur Bekämpfung von Schädlingen aus den Klassen Insekten, Arachnoidea und Nemathelminthes eignen.

## Beispiel 1

In einem 2-Liter-Kolben werden 300 ml Isopropanol, 300 ml $H_2O$ und 200 g 2,3-Dichlor-thiophen-4-aldehyd vorgelegt und innerhalb von 45 Minuten portionsweise mit 16 g $NaBH_4$ versetzt. Die Reaktion verläuft exotherm. Sie wird unter Kühlung bei 35 bis 40°C durchgeführt. Anschließend wird die zweiphasige Mischung 30 Minuten bei dieser Temperatur nachgerührt und mit 300 ml $H_2O$ und 500 ml $CH_2Cl_2$ versetzt. Nach der Phasentrennung wird die wäßrige Phase zweimal mit 250 ml $CH_2Cl$ nachextrahiert. Die organischen Phasen werden mit 250 ml $H_2O$ gewaschen und eingeengt. Der ölige Rückstand wird in Cyclohexan aufgenommen, worauf 182 g 2,3-Dichlor-4-hydroxymethyl-thiophen kristallin erhalten werden (87,3% d. Th.) Smp. 63—65°C.

## Beispiel 2

Analog Beispiel 1 wird hergestellt:

2,3-Dibrom-4-hydroxymethyl-thiophen
Smp. 79—82°C   Ausbeute 83%

Beispiel 3

Eine Mischung von 357 g $SOCl_2$ und 150 ml $CHCl_3$ wird in einem 1-Liter-Kolben vorgelegt, auf 10°C abgekühlt und innerhalb 35 Minuten bei 10 bis 15°C mit einer Lösung von 238 g 2,3-Dichlor-4-hydroxymethyl-thiophen in 350 ml Chloroform versetzt. Nach dem Abklingen der exothermen Reaktion wird die Lösung 1 Stunde bei 30°C nachgerührt, im Vakuum eingeengt und nach Zugabe von 0,5 g Triäthylamin destillativ bei 66 bis 69°C/20 Pa gereinigt. Die Ausbeute beträgt 192 g, d. h. 70% d. Th., 2,3-Dichlor-4-chlormethyl-thiophen. Analog wird hergestellt:

2,3-Dibrom-4-chlormethyl-thiophen
Sdp. 141 – 146°C/160 Pa    Ausbeute 27%

Beispiel 4

In einem 1-Liter-Kolben wird eine Mischung von 549 g 2,3-Dichlor-4-hydroxymethyl-thiophen und 450 ml $CHCl_3$ vorgelegt, auf 0°C abgekühlt und bei 0 bis 5°C innerhalb 1 Stunde mit einer Mischung von 271 g $PBr_3$ und 50 ml $CHCl_3$ versetzt. Die Lösung wird 1 Stunde nachgerührt und anschließend auf Eis gegeben. Nach dem Abtrennen der organischen Phase wird 2mal mit wenig Äther nachextrahiert. Die organischen Phasen werden unter vermindertem Druck eingeengt und der Rückstand mit 0,5 g Triäthylamin bei 58 – 60°C/13,3 Pa destilliert. Da die Destillationsverluste mit ca. 50% recht groß sind, wurde bei Folgeumsetzungen das Rohprodukt, 2,3-Dichlor-4-brommethyl-thiophen, ca. 90%ig, direkt verwendet.

Analog wird hergestellt:

2,3-Dibrom-4-brommethyl-thiophen
Rohprodukt    Ausbeute 96%

Beispiele für die Weiterverarbeitung zu insektiziden Phosphorsäureestern:

Beispiel I

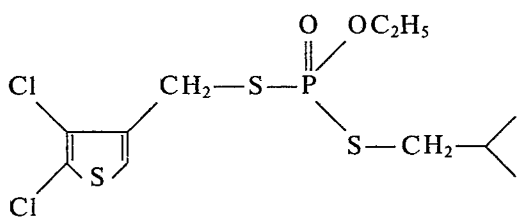

0,05 Mol 2,3-Dichlor-4-chlormethylthiophen werden in 100 ml Acetonitril vorgelegt und mit 0,05 Mol O-Äthyl-S-isobutyldithiophosphorsaurem Dimethylammoniumsalz in 100 ml Acetonitril versetzt. Nach 2 Stunden Rühren bei Zimmertemperatur wird noch 2 Stunden auf 50°C erwärmt, dann wird das Lösungsmittel entfernt, mit Äther aufgenommen und mit Wasser ausgeschüttelt, getrocknet und eingeengt. Man erhält 13,7 g O-Äthyl-S-isobutyl-S-(2,3-dichlor-thienyl-4-methyl)-dithiophosphat, entsprechend einer Ausbeute von 72%. Die leicht verunreinigte Probe wird über eine Säule mit Kieselgel (n-Hexan : Aceton = 9 : 1) gegeben.
$n_D^{25} = 1.5712$.

Analyse:
theor. C 34,8 H 4,5 S 25,3 Cl 18,7 P 8,2
gef. C 35,0 H 4,5 S 25,1 Cl 19,1 P 7,7

Analog dem angegebenen Beispiel werden die folgenden Verbindungen erhalten:

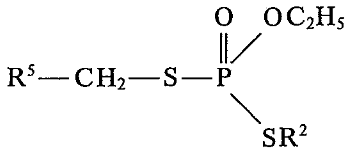

| | $R^5$ | $R^2$ | $n_D^{25}$ |
|---|---|---|---|
| II | 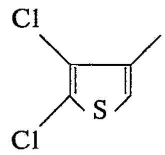 | n-$C_3H_7$ | 1,5703 |
| III | 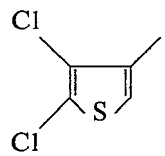 | 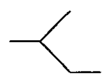 | ./. |
| IV | 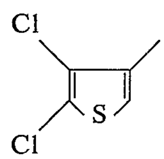 | 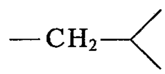 | 1,5712 |
| V | 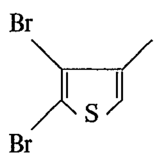 | 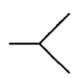 | 1,6018 |
| VI | 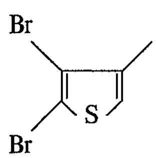 | 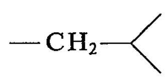 | 1,5888 |
| VII | 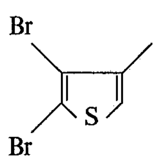 | 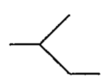 | 1,5968 |

Die angegebenen Phosphorsäureester sind geeignet, um schädliche oder lästige Gliedertiere aus der Klasse der Insekten, der Arachnoidea und den Nemathelminthes wirksam zu bekämpfen. Sie können auch mit Erfolg im Pflanzenschutz sowie auf dem Hygiene-, Vorratsschutz- und Veterinärsektor als Schädlingsbekämpfungsmittel eingesetzt werden.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die verkaufsfertigen Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen, z. B. Spritzbrühen, können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit über 95 Gew.-% Wirkstoff oder sogar den 100%igen Wirkstoff allein auszubringen.

Die folgenden Beispiele belegen die biologische Wirkung. Vergleichsmittel sind das aus der DE-PS 819 998 bekannte O,O-Dimethyl-S-(N-methyl-carbamoylmethyl)-phosphordithioat ($V_1$) und die strukturell ähnlichen Verbindungen O,O-Diäthyl-S-(thienyl-3-methyl)-dithiophosphat ($V_2$) und O,O-Diäthyl-S-(2,3-dichlor-thienyl-4-methyl)-dithiophosphat ($V_3$), die von der allgemeinen Formel in US-A-3 205 238 umfaßt sind. Die Numerierung der Wirkstoffe entspricht den Herstellungsbeispielen und der tabellarischen Aufzählung.

## Beispiel A

### Wirkung gegen Spinnmilben (Tetranychus telarius)

Getopfte Buschbohnen, die das zweite Folgeblattpaar entwickelt haben und einen starken Befall durch die Spinnmilbe Tetranychus telarius aufweisen, werden in der Spritzkabine allseitig mit der wäßrigen Wirkstoffaufbereitung tropfnaß gespritzt. Anschließend stehen die Pflanzen unter Gewächshausbedingungen bei 22 bis 24° C. Nach 6 Tagen wird die Wirkung beurteilt.

| Wirkstoff Nr. | Mortalitätsrate [%] von 0,1% | 0,05% | bei Wirkstoffkonzentrationen 0,02% | 0,01% |
|---|---|---|---|---|
| I | | 100 | 100 | |
| III | | 100 | 100 | 100 |
| $V_1$ | | Grenze der Wirkung | | |
| $V_2$ | 80 | unwirksam | | |
| $V_3$ | unwirksam | | | |

## Beispiel B

### Kontaktwirkung auf Moskito-Larven (Aedes aegypti)

200 ml Leitungswasser werden mit der Wirkstoffaufbereitung versetzt und darauf mit 30 bis 40 Moskito-Larven im 4. Larvenstadium besetzt.

Die Versuchstemperatur beträgt 20° C. Nach 24 Stunden wird die Wirkung ermittelt.

| Wirkstoff Nr. | Mortalitätsrate [%] von 1 ppm | 0,5 ppm | bei Wirkstoffkonzentrationen 0,25 ppm | 0,1 ppm |
|---|---|---|---|---|
| III | 100 | 100 | 100 | |
| $V_1$ | ca. 20 | | | |
| $V_2$ | unwirksam | | – | |
| $V_3$ | unwirksam | | | |

## Beispiel C

### Zuchtversuch mit Stubenfliegen-Larven (Musca domestica)

50 g eines Nährbodens aus Bäckerhefe, Trockenmilch, Wasser und Agar werden in warmem Zustand gründlich mit der wäßrigen Wirkstoffaufbereitung gemischt.

Nach dem Erkalten belegt man den Nährboden mit ca. 0,1 ml Fliegeneiern und beobachtet deren Entwicklung über eine Woche.

| Wirkstoff Nr. | Mortalitätsrate [%] von 50 ppm | 25 ppm | bei Wirkstoffkonzentrationen 10 ppm | 5 ppm |
|---|---|---|---|---|
| I | | 100 | 80 | |
| III | | 100 | 100 | |
| $V_2$ | unwirksam | | | |
| $V_3$ | unwirksam | | | |

## Beispiel D

### Kontaktwirkung auf Blattläuse (Aphis fabae); Spritzversuch

Getopfte Bohnenpflanzen (Vicia faba) mit starken Blattlauskolonien werden in der Spritzkammer mit den wäßrigen Wirkstoffaufbereitungen tropfnaß gespritzt.

Die Auswertung erfolgt nach 24 Stunden.

| Wirkstoff Nr. | Mortalitätsrate [%] von 0,1 | 0,05 | bei Wirkstoffkonzentrationen 0,02 | 0,01 |
|---|---|---|---|---|
| I | | 100 | 90 | |
| $V_1$ | unwirksam | | | |
| $V_2$ | | unwirksam | | |

## Beispiel E

### Wirkung auf Wurzelgallennemathoden (Meloidogyne incognita)

Junge Tomatenpflanzen werden in je 500 g Komposterde gepflanzt, die stark mit Wurzelgallennemathoden infiziert ist.

Nach 3 Tagen erfolgt die Behandlung mit der wäßrigen Wirkstoffaufbereitung durch Gießen von 30 ml Aufbereitung.

Nach 6 bis 8 Wochen bonitiert man die Wurzeln auf Gallenbildung.

| Wirkstoff Nr. | Wirkstoffkonzentration % |
|---|---|
| III | 0,05 keine Gallenbildung |
| $V_1$ | 0,1 unwirksam |
| $V_2$ | 0,1 unwirksam |

Beispiele für die Weiterverarbeitung zur Herstellung von N-Arylsulfonylpyrrolen, die als Diuretika verwendet werden können:

I. Durch Umsetzung von 3-Amino-4-phenoxy-5-sulfamoylbenzoesäure mit 2,3-Dibrom-4-brommethyl-thiophen in abs. Äthanol und anschließender Verseifung erhält man nach literaturbekannter Vorschrift (P. W. Feit, J. Med. Chem. 14, 432 [1971]) eine Verbindung der Formel

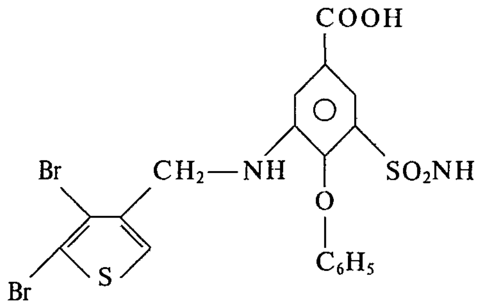

vom Fp. 241 – 242° C.

$C_{18}H_{14}O_5N_2S_2Br_2$ M = 562

Analyse:
ber.: C 38,4 H 2,5 O 14,2 M 5,0 S 11,4
gef. C 38,4 H 2,8 O 14,0 N 4,9 S 11,1

II. Die Weiterverarbeitung zum diuretisch wirkenden Endprodukt erfolgt durch Umsetzung mit 2,5-Dimethoxytetrahydrofuran nach der folgenden allgmeinen Vorschrift:
Eine Mischung von 15 ml Eisessig, 1 mMol der gemäß I. erhaltenen Verbindung und 1,5 mMol 2,5-Dimethoxytetrahydrofuran wird unter Rückfluß gekocht. Durch Entnahme von Proben wird die Beendigung der Reaktion dünnschichtchromatografisch überprüft. Das Reaktionsgemisch wird unter vermindertem Druck fast bis zur Trockene eingeengt und der Rückstand auf ca. 20 ml Eiswasser gegeben. Das ausgeschiedene Rohprodukt wird abgesaugt und getrocknet. In den Fällen, bei denen das Produkt in öliger Form anfällt, wird die wäßrige Phase mit Essigester extrahiert. Die Essigesterphase wird mit gesättigter Kochsalzlösung gewaschen, über Natriumsulfat getrocknet und unter vermindertem Druck zur Trockene eingeengt. Je nach Reinheit des Rohproduktes wird die erhaltene Verbindung durch Umkristallisation aus Äthanol oder Essigester/n-Hexan oder durch Säulenchromatographie an Kieselgel mit dem Laufmittel Methylenchlorid/Essigester isoliert. Die Ausbeuten liegen zwischen 40 und 96% der Verbindung der Formel

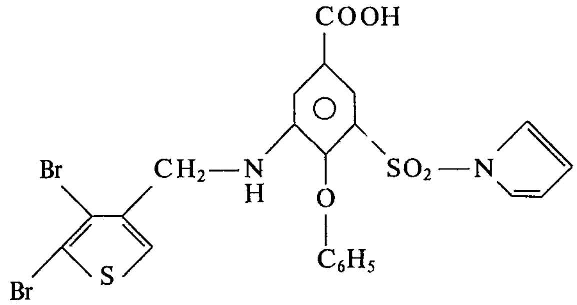

vom Fp. 229 – 230° C.

$C_{22}H_{16}O_5N_2S_2Br_2$ M = 612

Analyse:
ber.: C 43,2 H 2,6 N 4,6 O 13,1 S 10,5 Br 26,1
gef.: C 43,6 H 2,9 N 4,6 O 13,2 S 10,0 Br 25,4

**Patentansprüche**

1. Verbindungen der Formel I

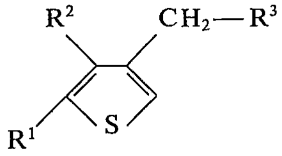

(I)

in der $R^1$ und $R^2$ jeweils gleich und Chlor- oder Bromatome und $R^3$ eine Hydroxygruppe oder ein Chlor- oder Bromatom bedeuten.

2. Verfahren zur Herstellung von Verbindungen der Formel I, dadurch gekennzeichnet, daß man einen Thiophenaldehyd der Formel II

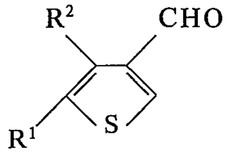

(II)

in der $R^1$ und $R^2$ die in Anspruch 1 angegebenen Bedeutungen haben, mit einem komplexen Hydrid reduziert und gegebenenfalls die erhaltene Hydroxymethylverbindung mit einem Säurechlorid oder Säurebromid einer Schwefel- oder Phosphorsäure oder mit konzentrierter Chlorwasserstoff- oder Bromwasserstoffsäure umsetzt.

**Claims**

1. A compound of the formula I

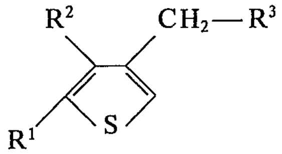

(I)

where $R^1$ and $R^2$ are identical and each denotes chlorine or bromine and $R^3$ is hydroxyl, chlorine or bromine.

2. A process for the preparation of a compound of the formula I, characterized in that a thiophenaldehyde of the formula II

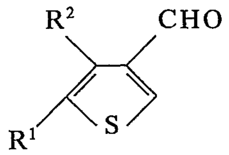

(II)

where $R^1$ and $R^2$ have the meanings given in claim 1, is reduced with a complex hydride and, where desired, the resulting hydroxymethyl compound is reacted with a sulfuric acid chloride or bromide or phosphoric acid chloride or bromide or with concentrated hydrochloric acid or hydrobromic acid.

**Revendications**

1. Composés de la formule I

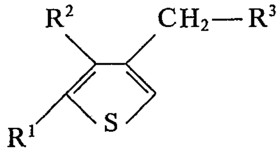

(I)

dans laquelle $R^1$ et $R^2$ sont identiques et désignent des atomes de chlore ou des atomes de brome et $R^3$ désigne un groupe oxhydryle ou un atome de chlore ou de brome.

2. Procédé de préparation de composés de la formule I, caractérisé en ce que l'on réduit un thiophènealdéhyde de la formule II

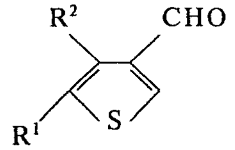

(II)

dans laquelle $R^1$ et $R^2$ possèdent les significations définies dans la revendication 1, par un hydrure complexe et l'on fait réagir, le cas échéant, le dérivé hydroxyméthylé obtenu avec un chlorure ou bromure de l'acide sulfurique ou de l'acide phosphorique ou avec de l'acide chlorhydrique ou de l'acide bromhydrique concentré.